# FASCICULE DE BREVET EUROPEEN

(11) **EP 0 400 735 B1**
(45) Date de publication et mention de la délivrance du brevet: **22.12.1993**
(21) Numéro de dépôt: 90201321.8
(22) Date de dépôt: 25.05.1990
(51) Int. Cl.: C07C 21/06, C07C 17/38

(54) **Procédé d'épuration de chlorure de vinyle**
Verfahren zur Reinigung von Vinylchlorid
Process for the purification of vinyl chloride

(30) Priorité: 02.06.1989 FR 8908234
(43) Date de publication de la demande: 05.12.1990
(73) Titulaire: SOLVAY (Société Anonyme), B-1050 Bruxelles (BE)
(72) Inventeur: Strebelle, Michel, B-1150 Bruxelles (BE)
(74) Mandataire: Nichels, William

(56) Documents cités:
- GB-A- 1 406 502
- US-A- 2 356 562

## Description

La présente invention concerne un procédé d'épuration de chlorure de vinyle en composés du type ester et plus particulièrement en composés esters tels que l'acétate de vinyle possédant une insaturation dans la molécule.

Il est connu que le chlorure de vinyle utilisé dans l'industrie est un composé qui peut être obtenu selon diverses méthodes de fabrication qui ont été optimalisées de façon à engendrer un composé dont la pureté est élevée et en tous cas suffisante pour les utilisations auxquelles ce composé est habituellement destiné et tout particulièrement à son homopolymérisation.

Un tout autre problème, apparu récemment dans l'industrie, pour des raisons économiques et d'environnement, est le problème posé par l'épuration de chlorure de vinyle résiduaire, non transformé ou issu de procédés industriels dans lesquels il a été mis en oeuvre. Dans ce contexte, le problème posé par la séparation du chlorure de vinyle, d'esters tels que l'acétate de vinyle, occupe une place particulière puisqu'il concerne la séparation de deux comonomères résultant d'une opération de copolymérisation dans laquelle, faute de solution satisfaisante au niveau du recyclage des deux comonomères, on acceptait de perdre tout ou partie des deux monomères par des opérations de destruction non sélective telles que le brûlage.

On a maintenant trouvé un nouveau procédé qui ne possède pas les inconvénients précités et qui permet, moyennant la destruction du composé ester, de récupérer du chlorure de vinyle très pur, ce qui permet d'en faire une utilisation identique à celle d'un chlorure de vinyle directement issu d'une unité de fabrication de monomères.

La présente invention concerne à cet effet un procédé d'épuration de chlorure de vinyle en composés de type ester présentant une structure d'esters d'énol, caractérisé en ce que le chlorure de vinyle subit un traitement comprenant une étape de lavage alcalin suivie d'une étape de traitement avec un bisulfite à un pH de 5 à 9.

Les composés de type ester présentant une structure d'esters d'énol, présents généralement dans le chlorure de vinyle sont des composés aptes à copolymériser par voie radicalaire avec le chlorure de vinyle. Ces composés du type esters d'énol donnent par hydrolyse des dérivés carbonylés cétoniques ou aldéhydiques aptes à former des composés d'addition avec un bisulfite, soit des aldéhydes en général, soit des méthylcétones et des cétones cycliques. Un composé de ce type ayant conduit à de bons résultats est l'acétate de vinyle. Il est toutefois évident que le procédé de l'invention s'applique aisément à tout type d'ester insaturé présentant une structure d'ester d'énol qui peut se retrouver pour une quelconque raison dans le chlorure de vinyle.

L'étape de lavage alcalin du chlorure de vinyle impur peut être réalisée en principe avec tout agent basique connu, mais est habituellement réalisée avec une solution aqueuse d'une base dérivée de métaux alcalins ou alcalino-terreux. De préférence, ce traitement est effectué avec un hydroxyde de ces métaux. Enfin, de bons résultats ont été obtenus avec de l'hydroxyde de sodium. Quoique la présente invention n'entend en aucune façon être limitée ou être tributaire d'une quelconque explication ou théorie scientifique, il est fort probable que le traitement alcalin transforme l'entièreté du composé ester en un sel et un aldéhyde. Ainsi, plus particulièrement dans le cas de l'acétate de vinyle, il se formerait à ce stade de l'acétate de sodium et de l'acétaldéhyde lorsque le procédé est exécuté à l'intervention d'hydroxyde de sodium.

Lorsque le lavage alcalin est effectué avec une solution aqueuse d'hydroxyde de sodium, la concentration en NaOH est généralement comprise entre 0,01 et 10 M/l et de préférence entre 0,1 M/l et 2 M/l.

L'étape de lavage alcalin est suivie d'une étape de traitement du chlorure de vinyle avec un bisulfite, ce qui a comme conséquence d'épurer le chlorure de vinyle probablement par élimination d'aldéhydes, tels que l'acétaldéhyde résiduaire, présents dans le milieu. Parmi les bisulfites, on opère généralement avec des bisulfites de métaux alcalins; de bons résultats ont été observés avec le bisulfite de sodium.

Les bisulfites de l'invention peuvent être obtenus par tout procédé connu tel que l'addition d'eau à du métabisulfite ou l'addition d'acide à des sulfites.

Lorsque le traitement est effectué avec le bisulfite de sodium, on opère avantageusement sous pH contrôlé et, dans tous les cas, situé entre des valeurs de 5 et 9 afin d'éviter la formation de quantités trop élevées de SO₂ dans le milieu de réaction. De préférence, le pH du milieu sera maintenu entre des valeurs comprises entre 6,5 et 8,5.

Le contrôle du pH peut être assuré par tout moyen permettant d'atteindre cet effet. Un moyen ayant donné de bons résultats consiste à ajouter du sulfite de sodium en quantités suffisantes pour atteindre le pH désiré.

Lorsque le traitement est effectué avec une solution aqueuse de bisulfite de sodium, la concentration en NaHSO₃ est généralement comprise entre 0,01 M/l et la limite de solubilité dans l'eau, dans les conditions de température et de pression considérées, de ce composé. De préférence, la concentration est toutefois comprise entre 0,05 et 1 M/l.

Le traitement au bisulfite peut être effectué par une solution aqueuse contenant uniquement le bisulfite ou peut être réalisé en présence d'autres composants n'affectant pas le bon déroulement de la réaction. Ainsi, une exécution ayant donné de bons résultats consiste à combiner l'opération de séchage du chlorure de vinyle avec celle du traitement au bisulfite. Pour ce faire, on opère de préférence avec des mélanges de NaCl et de NaHSO₃ dont le point de congélation est suffisamment bas pour assurer simultanément l'élimination de l'acétaldéhyde et le séchage du chlorure de vinyle. Lors d'une telle opération, le traitement est avantageusement réalisé dans un scrubber de séchage maintenu aux environs de -10°C.

Outre les étapes précitées, dont la séquence opérationnelle est essentielle pour l'obtention de chlorure de vinyle exempt d'esters, le procédé de l'invention peut prévoir d'autres étapes selon les conditions particulières qui ont conduit à un tel chlorure de vinyle.

Le procédé de l'invention, lorsqu'il est réalisé à l'intervention de bisulfite de sodium sur du chlorure de vinyle issu d'une fabrication de copolymérisation de chlorure de vinyle et d'acétate de vinyle, peut être effectué dans des conditions de température comprises habituellement entre -20°C et 50°C et, de préférence, entre 0° et 10°C pour ce qui concerne l'étape de lavage alcalin et entre -15° et 40°C et, de préférence, entre -10° et 35°C pour ce qui concerne l'étape au bisulfite.

En ce qui concerne les conditions de pression, celles-ci ne sont pas critiques et sont généralement comprises entre 1 et 10 bars pour les deux étapes. Habituellement, les deux étapes sont réalisées à la pression atmosphérique.

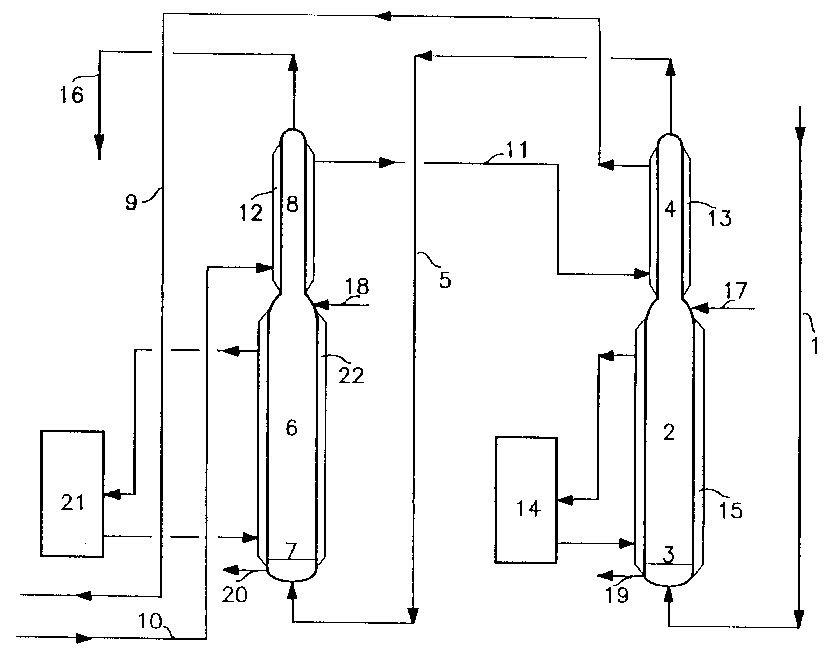

Le procédé de l'invention peut être réalisé dans toute installation permettant d'effectuer les étapes décrites ci-dessus et comportant des colonnes noyées, arrosées ou à garnissage capables de fonctionner soit en batch, soit en continu. Un schéma d'une installation ayant donné de bons résultats est repris à la figure 1 et comprend :
- une conduite 1 par laquelle est introduit dans le système du chlorure de vinyle gazeux contenant l'ester à éliminer,
- un premier réacteur 2, équipé d'une double enveloppe 15, dans lequel est introduit le chlorure de vinyle impur via la conduite 1. Ce réacteur 2 est avantageusement équipé d'un dispositif de répartition des gaz dans le liquide 3 et d'un condenseur 4 permettant respectivement d'effectuer un bon barbotage des gaz et de retenir les produits liquides à la température de lavage alcalin,
- une conduite 5 reliant le condenseur 4 du réacteur 2 à un deuxième réacteur 6 destiné à effectuer le traitement au bisulfite,
- ce réacteur 6 est de nouveau avantageusement équipé d'un dispositif de répartition des gaz dans le liquide 7 et d'un condenseur 8 dont les fonctions sont identiques à celles du dispositif 3 et du condenseur 4,
- le chlorure de vinyle pur est ensuite envoyé par la conduite 16 au séchage suivi d'une recompression et d'une liquéfaction afin de permettre le stockage du chlorure de vinyle pur,
- enfin, l'installation est complétée par un circuit de réfrigération permettant respectivement via les conduites 10 et 11 de refroidir par les doubles enveloppes 12 et 13 les condenseurs 4 et 8, et par des circuits de réfrigération indépendant 14 et 21 permettant d'assurer le refroidissement de la double enveloppe 15 du réacteur de lavage alcalin 2 et de la double enveloppe 22 du réacteur 6.

Les réacteurs 2 et 6 peuvent en outre être équipés pour fonctionner en continu ou en discontinu. A cet effet, des conduites 17 et 18 sont prévues pour les alimenter respectivement en solutions aqueuses de NaOH et de bisulfite. Par ailleurs, afin de maintenir un niveau constant dans les réacteurs 2 et 6, ceux-ci comportent également des conduites de "purge" 19 et 20 qui permettent d'éliminer les solutions aqueuses partiellement épuisées.

L'installation peut être réalisée entièrement ou partiellement en tout matériau approprié, recouvert ou non de composés polymériques permettant d'améliorer la résistance des matériaux à certains des réactifs mis en oeuvre. Habituellement, la totalité de l'installation est réalisée en acier inoxydable.

Le chlorure de vinyle récupéré à la sortie du deuxième réacteur peut être utilisé dans toute fabrication et plus particulièrement pour la fabrication d'homopolymères à grande pureté.

Enfin, bien que l'invention concerne l'épuration de chlorure de vinyle en composés de type ester présentant une structure d'ester d'énol, il est évident qu'elle peut également convenir sans autre modification fondamentale à l'épuration d'autres monomères du même type tels que le chlorure de vinylidène, fluorure de vinyle et fluorure de vinylidène ainsi qu'à l'épuration de ces composés en certaines impuretés cétoniques ou aldéhydiques susceptibles de réagir avec le bisulfite et dont l'origine n'est pas l'ester d'énol.

L'invention est illustrée par les exemples suivants.

### Exemple 1

On opère dans une installation telle qu'illustrée à la figure 1 dans laquelle la colonne 2 et la colonne 6 ont un diamètre de 65 mm, une hauteur de 550 mm et un volume en liquide de 400 cm³.

La colonne 2, maintenue par refroidissement à une température de 6°C, contient une solution aqueuse de NaOH 2 molaire et la composition du pied de la colonne 6, maintenue à 25°C, est constituée d'une solution aqueuse de NaHSO₃ 0,1 M.

On introduit dans cette installation, via la conduite 1 avec un débit de gaz d'environ 145 lN/h (litres normaux mesurés à 0°C et à pression atmosphérique/heure), du chlorure de vinyle récupéré d'une installation de copolymérisation avec de l'acétate de vinyle contenant 2.500 ppm vol d'acétate de vinyle pendant une durée totale d'essai de 6 h.

Les résultats suivants ont été observés :
- Entrée du réacteur à NaHSO₃ :
   - Acétate de vinyle en ppm vol: < 1
   - Acétaldéhyde en ppm vol: 250
- Sortie du réacteur à NaHSO₃ :
   - Acétate de vinyle en ppm vol: < 1
   - Acétaldéhyde en ppm vol: < 1
   - SO₂ en ppm vol: 60

On peut conclure, au vu des résultats ci-dessus, que le procédé de l'invention permet d'éliminer d'une façon quantitative tout l'acétate de vinyle présent dans le chlorure de vinyle et que celui-ci a dès lors une pureté permettant son recyclage. On peut également conclure de ces résultats que, lorsque le traitement au bisulfite est effectué sans contrôle du pH, il y a formation de quantités de SO₂ mesurables.

### Exemple 2

On opère dans des conditions identiques à celles de l'exemple 1 mais en contrôlant le pH par introduction de NaSO₃ en une quantité telle que le pH se situe tout au long de l'expérience à une valeur de 5,5.

On observe dans ces conditions les mêmes résultats que dans l'exemple 1 à l'exception du seul résultat en SO₂ qui tombe à 3,5 ppm vol.

### Exemple 3

On opère dans les conditions de l'exemple 1 mais en introduisant suffisamment de NaSO₃ pour pouvoir travailler tout au long de l'expérience à un pH de 8 et on observe que, dans ces conditions, tout autre résultat étant conservé, la quantité de SO₂ formée est inférieure à 0,1 ppm vol.

On peut dès lors conclure des exemples que le procédé de l'invention, exécuté sans aucune précaution particulière (exemple 1), permet de détruire la totalité de l'acétate de vinyle et que, lorsque ce procédé est effectué sous contrôle de pH (exemples 2 et 3), il permet de minimiser voire d'éviter toute formation de SO₂.

## Revendications

1. Procédé d'épuration de chlorure de vinyle en composés de type ester insaturé présentant une structure d'ester d'énol, caractérisé en ce que le chlorure de vinyle impur subit un traitement comprenant une étape de lavage alcalin suivie d'une étape de traitement avec un bisulfite à un pH de 5 à 9.

2. Procédé selon la revendication 1, caractérisé en ce que l'étape de lavage alcalin est réalisée avec une solution aqueuse d'une base dérivée de métaux alcalins ou alcalino-terreux.

3. Procédé selon l'une des revendications 1 ou 2, caractérisé en ce que le bisulfite mis en oeuvre est un bisulfite de métal alcalin.

4. Procédé selon la revendication 3, caractérisé en ce que le bisulfite mis en oeuvre est du bisulfite de sodium.

5. Procédé selon la revendication 4, caractérisé en ce que l'étape de lavage alcalin est suivie d'une étape de traitement au bisulfite de sodium sous contrôle du pH entre des valeurs comprises entre 6,5 et 8,5.

6. Procédé selon l'une quelconque des revendications 1 à 5, caractérisé en ce que l'ester présent dans le chlorure de vinyle impur est l'acétate de vinyle.

## Patentansprüche

1. Verfahren zur Reinigung von Vinylchlorid von Verbindungen vom ungesättigten Estertyp, die eine Enolester-Struktur besitzen, dadurch gekennzeichnet, daß das verunreinigte Vinylchlorid einer Behandlung unterworfen wird umfassend eine alkalische Waschstufe gefolgt von einer Behandlung mit Bisulfit bei einem pH von 5-9.

2. Verfahren nach Anspruch 1, dadurch gekennzeichnet, daß die alkalische Waschstufe mit einer wässrigen Lösung einer Alkali- oder Erdalkalibase durchgeführt wird.

3. Verfahren nach Anspruch 1 oder 2, dadurch gekennzeichnet, daß das eingesetzte Bisulfit ein Alkalimetallbisulfit ist.

4. Verfahren nach Anspruch 3, dadurch gekennzeichnet, daß das eingesetzte Bisulfit Natriumbisulfit ist.

5. Verfahren nach Anspruch 4, dadurch gekennzeichnet, daß die alkalische Waschstufe von einer Behandlungsstufe mit Natriumbisulfit unter Kontrolle des pH-Wertes im Bereich von 6,5 bis 8,5 gefolgt ist.

6. Verfahren nach einem der Ansprüche 1-5, dadurch gekennzeichnet, daß der in dem verunreinigten Vinylchlorid enthaltene Ester Vinylacetat ist.

## Claims

1. Process for the purification of vinyl chloride from compounds of the unsaturated ester type exhibiting an enol ester structure, characterized in that the impure vinyl chloride undergoes a treatment comprising an alkaline washing stage followed by a stage of treatment with a bisulphite at a pH of 5 to 9.

2. Process according to Claim 1, characterized in that the alkaline washing stage is carried out with an aqueous solution of a base derived from alkali or alkaline-earth metals.

3. Process according to either of Claims 1 and 2, characterized in that the bisulphite used is an alkali metal bisulphite.

4. Process according to Claim 3, characterized in that the bisulphite used is sodium bisulphite.

5. Process according to Claim 4, characterized in that the alkaline washing stage is followed by a stage of treatment with sodium bisulphite with control of the pH between values included between 6.5 and 8.5.

6. Process according to any one of Claims 1 to 5, characterized in that the ester present in the impure vinyl chloride is vinyl acetate.
